# EUROPÄISCHE PATENTANMELDUNG

(11) **EP 3 349 028 A1**
(43) Veröffentlichungstag der Anmeldung: **18.07.2018**
(21) Anmeldenummer: 17151483.9
(22) Anmeldetag: 13.01.2017
(51) Int. Cl.: G01R 33/28, G01R 33/38, G01R 33/385, G01R 33/381, A61B 5/055

(54) **MRT-VORRICHTUNG UND VERFAHREN ZUR VERMESSUNG EINES KOPFBEREICHS EINES PATIENTEN**

(71) Anmelder: Sirona Dental Systems GmbH, 64625 Bensheim (DE)
(72) Erfinder: Hell, Erich, 66557 Wustweiler (DE); Rasche, Volker, 89155 Erbach (DE); Ulrici, Johannes, 64285 Darmstadt (DE)
(74) Vertreter: Sommer, Peter

(57) **Zusammenfassung**

Die Erfindung betrifft MRT-Vorrichtung (1) zur Vermessung eines Kopfbereichs (2) eines Patienten (3), umfassend mindestens eine Hauptmagnetfeldeinheit (4), mindestens eine Gradientenspulenanordnung (5), eine geschlossene Patientenöffnung (7), mindestens eine Anregungsspule (6) und mindestens eine Empfangsspule. Eine geschlossene Patientenöffnung (7) der MRT-Vorrichtung (1) weist dabei in einem ersten Bereich (8) einen ersten Innendurchmesser (9) senkrecht zu einer Mittelachse (12) der MRT-Vorrichtung (1) und im zweiten Bereich (10) einen zweiten Innendurchmesser (11) auf, der parallel zum ersten Innendurchmesser (9) angeordnet ist. Der erste Innendurchmesser (9) ist kleiner als der zweite Innendurchmesser (11), wobei der erste Bereich (8) zumindest teilweise den Kopfbereich (2) des Patienten umfasst, wobei der zweite Bereich (10) zumindest teilweise einen Torsobereich (14) des Patienten umfasst. Die MRT-Vorrichtung (1) ist schräg angeordnet, wobei die Mittelachse (12) der MRT-Vorrichtung (1) in einer Z-Richtung relativ zu einer Richtung (15) der Erdgravitation einen Winkel (16) zwischen 20° und 75° aufweist.

## Beschreibung

### Technisches Gebiet

Die Erfindung betrifft eine MRT-Vorrichtung zur Vermessung eines Kopfbereichs eines Patienten, umfassend mindestens eine Hauptmagnetfeldeinheit, mindestens eine Gradientenspulenanordnung, eine Patientenöffnung, mindestens eine Anregungsspule und mindestens eine Empfangsspule.

### Stand der Technik

Aus dem Stand der Technik sind mehrere MRT-Vorrichtungen zur Vermessung eines Patienten bekannt.

DE 10 2009 027119 B4 offenbart ein MRT-System zur bildgebenden Erfassung eines Kopfbereichs mit einem Permanentmagneten, einer Gradientenspule und mindestens einer Hochfrequenzspule, wobei eine Magnetfeldeinheit an einem vertikal angeordneten Stativ in der Höhe verstellt werden kann. Zusätzlich kann die Magnetfeldeinheit bis zu einem Winkel von 45 ° relativ zur Längsachse des Stativs geschwenkt werden. Der Patient nimmt während der Vermessung eine sitzende Position ein, wobei der Kopf des Patienten durch eine Stirnstütze und Ohroliven relativ zum Stativ positioniert wird.

Bei diesem MRT-System wird der Patient auf eine aufwändige Art und Weise relativ zur MRT-Vorrichtung positioniert, indem der Patient in eine zylinderförmige Patientenöffnung eingebracht wird. Dabei werden ein höhenverstellbarer Hocker und das vertikal verstellbare Stativ des MRT-Systems manuell so eingestellt, dass der Kopf des Patienten vermessen werden kann.

DE 197 34 138 B2 offenbart eine MRT-Vorrichtung, umfassend eine Gradienten-Spulenanordnung und eine MRT-Hauptspule.

US 2013/0023418 A1 offenbart eine MRT-Vorrichtung mit einem Kühlungssystem aus einer ersten Stufe und einer zweiten Stufe.

Die Aufgabe der vorliegenden Erfindung besteht also darin, eine MRT-Vorrichtung bereitzustellen, die die Vermessung eines Kopfbereichs eines Patienten ermöglicht, wobei die Abmessungen der MRT-Vorrichtung möglichst kompakt sind und eine bequeme Positionierung des Patienten relativ zur MRT-Vorrichtung ermöglicht wird.

### Darstellung der Erfindung

Die Erfindung betrifft eine MRT-Vorrichtung zur Vermessung eines Kopfbereichs eines Patienten, umfassend mindestens eine Hauptmagnetfeldeinheit, mindestens eine Gradientenspulenanordnung, eine Patientenöffnung, mindestens eine Anregungsspule und mindestens eine Empfangsspule. Eine geschlossene Patientenöffnung der MRT-Vorrichtung weist dabei in einem ersten Bereich einen ersten Innendurchmesser senkrecht zu einer Mittelachse der MRT-Vorrichtung und im zweiten Bereich einen zweiten Innendurchmesser auf, der parallel zum ersten Innendurchmesser angeordnet ist, wobei der erste Innendurchmesser kleiner als der zweite Innendurchmesser ist. Der erste Bereich umfasst zumindest teilweise den Kopfbereich des Patienten, wobei der zweite Bereich zumindest teilweise einen Torsobereich des Patienten umfasst. Die MRT-Vorrichtung ist schräg angeordnet, wobei die Mittelachse der MRT-Vorrichtung relativ zu einer Richtung der Erdgravitation einen Winkel zwischen 20° und 75° aufweist.

Die MRT-Vorrichtung (Magnetresonanztomographie-Vorrichtung) ist eine herkömmliche MRT-Vorrichtung zur Vermessung eines Kopfes. Die MRT-Vorrichtung weist dabei ein Messvolumen auf, wobei das Objektvolumen des Objekts innerhalb des Messvolumens angeordnet wird, um das Objekt zu vermessen.

Bei einer herkömmlichen MRT-Vorrichtung erfolgt die Vermessung innerhalb des Objektvolumens durch Überlagerung des Hauptmagnetfeldes mit x-, y- und z- Gradientenfeldern, welche zeitlich variiert werden. Durch die Gradientenfelder kann das Magnetfeld im Objekt räumlich variiert werden, wodurch die Larmorfrequenz der Protonen im Objekt in gleichem Maße ortsabhängig wird. Hierdurch sind eine Ortskodierung der Anregung der Protonen mit einem von der Anregungsspule erzeugten Anregungspuls und/oder eine Ortskodierung des von den Protonen emittierten und von der Empfangsspule gemessenen Messsignals möglich.

Bei einer herkömmlichen MRT-Vorrichtung erfolgt eine Ortskodierung der Voxel des Messvolumens indem die ausgelesenen Signale den einzelnen Volumenelemente (Voxeln) zugeordnet werden, wobei mit linear ortsabhängigen Magnetfeldern (Gradientenfeldern) eine Ortskodierung erzeugt wird. Dabei wird ausgenutzt, dass für ein bestimmtes Teilchen die Larmorfrequenz von der magnetischen Flussdichte abhängt (je stärker der Feldanteil senkrecht zur Richtung des Teilchendrehimpulses, desto höher die Larmorfrequenz).

Eine Möglichkeit, die Gradientenfelder zur Ortskodierung zu nutzen, ist hierbei, dass bei der Anregung ein erster Gradient angelegt wird, so dass nur eine einzelne Schicht des Objekts die passende Larmorfrequenz besitzt, also nur die Spins dieser Schicht ausgelenkt werden (Schichtselektionsgradient).

Ein zweiter Gradient quer zum ersten wird nach der Anregung kurz eingeschaltet und bewirkt eine kontrollierte Dephasierung der Spins dergestalt, dass in jeder Bildzeile Spins mit einer anderen Phasenlage präzedieren (Phasenkodiergradient).

Der dritte Gradient wird während der Messung in einer zu den beiden anderen linear unabhängigen Richtungen geschaltet. Der dritte Gradient sorgt dafür, dass die Spins jeder Bildspalte eine andere Präzessionsgeschwindigkeit haben, also eine andere Larmorfrequenz senden (Auslesegradient, Frequenzkodiergradient).

Alle drei Gradienten zusammen bewirken also eine Kodierung des Signals in drei Raumebenen. Das empfangene Signal gehört zu einer bestimmten Schicht des Objekts und enthält eine Kombination aus Frequenz- und Phasenkodierung, die der Computer mit einer Fourier-Transformation in ein zweidimensionales Bild umrechnen kann.

Die Gradientenspulenanordnung besteht also aus einer ersten X-Gradientenspule, einer zweiten Y-Gradientenspule und einer dritten Z-Gradientenspule, wobei die Gradientenachsen der Gradienten linear unabhängig zueinander angeordnet sind.

Die Hauptmagnetfeldeinheit erzeugt das Hauptmagnetfeld und kann beispielsweise aus mindestens einem Dauermagneten, mindestens einer Magnetspule oder mindestens einer supraleitenden Magnetspule bestehen.

Die Patientenöffnung kann beliebig geformt sein und beispielsweise eine kreisförmige Form, eine elliptische Form oder eine rechteckige Form aufweisen. Die Patientenöffnung muss so groß gestaltet sein, dass insbesondere der Kopfbereich des Patienten in den Messbereich der MRT-Vorrichtung hineinpasst.

Die Anregungsspule und die Empfangsspule können separate Spulen oder in einer einzelnen Radiofrequenzspule vereinigt sein.

Die Mittelachse der MRT-Vorrichtung kann beispielsweise mit einer Achse der im Wesentlichen zylindrischen Z-Gradientenspule übereinstimmen. Die Mittelachse kann auch mit einer Symmetrieachse der Patientenöffnung übereinstimmen. Die Mittelachse der MRT-Vorrichtung kann auch mit der Symmetrieachse des Hauptmagnetfeldes der Hauptmagnetfeldeinheit übereinstimmen bzw. parallel zu der Symmetrieachse des Hauptmagnetfeldes ausgerichtet sein.

Der erste Innendurchmesser des ersten Bereichs senkrecht zur Mittelachse ist parallel zum zweiten Innendurchmesser des zweiten Bereichs angeordnet. Der erste Innendurchmesser kann beispielsweise so groß gewählt sein, dass der Kopf des Patienten hineinpasst, wobei der zweite Innendurchmesser so gewählt werden kann, dass insbesondere der Schulterbereich oder der Torsobereich des Patienten hineinpasst.

Die MRT-Vorrichtung ist schräg angeordnet, wobei die Mittelachse der MRT-Vorrichtung relativ zu der Richtung der Erdgravitation ein Winkel zwischen 20° und 75° aufweist. Der Patient wird also in eine sitzende Position gebracht und schräg in die MRT-Vorrichtung hineingefahren.

Ein Vorteil der vorliegenden MRT-Vorrichtung besteht darin, dass ein schräg sitzender Patient auf eine komfortable Art und Weise auf dem Patientensitz positioniert werden kann und vollautomatisch in die MRT-Vorrichtung hineingefahren werden kann. Denn der Patient kann in einer für den Patienten bequemen nach hinten gelehnten, sitzenden Position auf einem Patientensitz positioniert werden und in die MRT-Vorrichtung hineingefahren werden.

Ein weiterer Vorteil der vorliegenden MRT-Vorrichtung besteht darin, dass durch die mehrstufige Gestaltung der Patientenöffnung die MRT-Vorrichtung kompakter realisiert werden kann. Denn der Schulterbereich und der Torsobereich des Patienten kann im zweiten Bereich mit größerem Innendurchmesser aufgenommen werden. Hierdurch können die Außenabmessungen der MRT-Vorrichtung kompakter und kürzer gestaltet werden, wobei die Größe der Patientenöffnung im Schulterbereich nicht eingeschränkt werden muss.

Ein weiterer Vorteil besteht darin, dass die Kollision einer unteren Fläche der MRT-Vorrichtung mit den Oberschenkeln oder den Knien des Patienten beim Hineinfahren verhindert wird, da zum einen die Knie durch die schräge Anordnung der MRT-Anordnung im Vergleich zu einer aufrecht sitzenden Patientenposition nicht so stark angewinkelt werden und bei gleichen Systemaußenabmessungen durch den größeren Innendurchmesser der Patientenöffnung unterhalb des Kopfes mehr Raum für Oberschenkel und Knie zur Verfügung stehen.

Vorteilhafterweise kann der zweite Innendurchmesser des zweiten Bereichs entlang einer coronal angeordneten Schulterachse des Torsobereichs des Patienten angeordnet sein.

Die Coronalebene ist eine Frontalebene und bezeichnet die bei einer Vorderansicht des Menschen sichtbare Bewegungsebene.

Der zweite Innendurchmesser des zweiten Bereichs ist also entlang der Schulterachse des Torsobereichs so groß gewählt, dass der Torsobereich auch eines größeren Patienten hineinpasst.

Vorteilhafterweise können ein erster Querschnitt der Patientenöffnung im ersten Bereich und/oder ein zweiter Querschnitt im zweiten Bereich eine kreisförmige Form, eine elliptische Form oder eine rechteckige Form aufweisen.

Dadurch werden der erste Querschnitt des ersten Bereichs und der zweite Querschnitt des zweiten Bereichs so gewählt, dass der Kopfbereich des Patienten in den ersten Bereich und der Torsobereich des Patienten in den zweiten Bereich hineinpassen.

Vorteilhafterweise kann die Patientenöffnung im ersten Bereich den ersten Innendurchmesser zwischen 250 mm und 650 mm und im zweiten Bereich den zweiten Innendurchmesser zwischen 500 mm und 800 mm aufweisen.

Durch die Auswahl dieser Abmessungen des ersten Innendurchmessers und des zweiten Innendurchmessers kann ein durchschnittlicher Patient in die Patientenöffnung hineingefahren werden.

Vorteilhafterweise kann die Gradientenspulenanordnung innerhalb der MRT-Vorrichtung um die Patientenöffnung angeordnet sein, wobei die Gradientenspulenanordnung im ersten Bereich der Patientenöffnung einen ersten Gradientenspulen-Innendurchmesser und im zweiten Bereich der Patientenöffnung einen zweiten Gradientenspulen-Innendurchmesser aufweisen, der parallel zum ersten Gradientenspulen-Innendurchmesser angeordnet ist, wobei der erste Gradientenspulen-Innendurchmesser kleiner als der zweite Gradientenspulen-Innendurchmesser ist.

Die Gradientenspulenanordnung kann also aus einer X-Gradientenspule, einer Y-Gradientenspule und einer Z-Gradientenspule bestehen, wobei die X-Gradientenspule und die Y-Gradientenspule als Sattelspulen ausgeführt sein können und wobei die Z-Gradientenspule zylinderförmig aufgewickelte Spulenwindungen aufweisen kann. Die Gradientenspulenanordnung kann also unmittelbar hinter der Verkleidung der Patientenöffnung angebracht sein.

Der erste Gradientenspulen-Innendurchmesser des ersten Bereichs ist also kleiner als der zweite Gradientenspulen-Innendurchmesser des zweiten Bereichs, wie bei der Patientenöffnung, so dass der Kopfbereich des Pateinten näher am ersten Bereich der Gradientenspulenanordnung positioniert wird und somit die Gradientenfelder der einzelnen Gradientenspulen bei gleicher Leistung der Spannungsversorgung stärker sind.

Vorteilhafterweise kann die Gradientenspulenanordnung nur im ersten Bereich, also im Kopfbereich des Patienten, angeordnet sein, so dass der zweite Bereich also der Torsobereich, frei von der Gradientenspulenanordnung ist.

Dadurch ist die Gradientenspulenanordnung nur im Kopfbereich des Patienten angeordnet, so dass die Vermessung des Patienten mittels der MRT-Vorrichtung nur im Kopfbereich des Patienten ermöglicht wird.

Vorteilhafterweise kann der erste Bereich der Patientenöffnung eine Länge von mindestens 150 mm aufweisen.

Dadurch ist die Länge des ersten Bereichs entlang der Mittelachse der MRT-Vorrichtung lang genug, um einen Kopfbereich aufzunehmen.

Vorteilhafterweise kann die Gradientenspulenanordnung eine Z-Gradientenspule für eine Z-Richtung aufweisen, wobei die Z-Gradientenspule Spulenwindungen aufweist, die zylinderförmig aufgewickelt sind, wobei der Abstand der Spulenwindungen zueinander über die Länge der Z-Gradientenspule in Z-Richtung variiert.

Dadurch variiert die Windungsdichte der zylindrisch aufgewickelten Spulenwindungen entlang der Z-Richtung des Hauptmagnetfeldes.

Vorteilhafterweise kann die Windungsdichte der Spulenwindungen der Z-Gradientenspule symmetrisch zu einer Ebene der Z-Gradientenspule ausgestaltet ist, die senkrecht zu der Mittelachse der MRT-Vorrichtung angeordnet ist.

Dadurch ist das Z-Gradientenfeld der Z-Gradientenspule ebenfalls symmetrisch zu dieser Ebene angeordnet.

Vorteilhafterweise kann die Windungsdichte der Spulenwindungen der Z-Gradientenspule asymmetrisch sein, wobei die Spulenwindungen der Z-Gradientenspule auf einer zu einem Torso des Patienten gerichteten Seite im Vergleich zu der gegenüberliegenden zum Kopf des Patienten gerichteten Seite eine höhere Windungsdichte aufweisen.

Durch die höhere Windungsdichte im Torsobereich kann ein Abstand zwischen einer unteren Fläche der Gradientenspulenanordnung im Torsobereich relativ zu einem Aufnahmezentrum im Vergleich zum Abstand der oberen Fläche der Gradientenspulenanordnung im Kopfbereich relativ zum Aufnahmezentrum kürzer ausgestaltet werden. Dadurch kann die MRT-Vorrichtung kompakter konstruiert werden.

Vorteilhafterweise kann zwischen dem ersten Bereich und dem zweiten Bereich der Patientenöffnung ein Übergang ausgebildet sein, der zwischen dem Kopfbereich des Patienten und dem Torsobereich des Patienten angeordnet ist.

Der Übergang zwischen dem ersten Bereich und dem zweiten Bereich kann beispielsweise die Form einer Stufe oder auch die Form eines schrägen Übergangs mit einem Winkel zwischen 30° und 60° relativ zur Z-MRT-Vorrichtung aufweisen. Vorteilhafterweise kann die MRT-Vorrichtung eine X-Gradientenspule und/oder eine Y-Gradientenspule aufweisen, die als Sattelspulen ausgeführt sein können.

Dadurch wird eine präzise Ortskodierung in einer X-Richtung und einer Y-Richtung ermöglicht.

Vorteilhafterweise kann die Hauptmagnetfeldeinheit mindestens eine supraleitende Magnetspule aufweisen, die mittels eines Kühlsystems gekühlt ist, welches für die Supraleitung der Magnetspule erforderlichen Temperaturen erzeugt.

Durch die supraleitende Magnetspule wird ein höheres Hauptmagnetfeld beispielsweise bis zu 11 Tesla ermöglicht.

Vorteilhafterweise kann das Kühlsystem einen Kryostaten mit Helium als Kühlmittel aufweisen.

Dadurch wird eine effiziente Kühlung des Kühlsystems ermöglicht.

Vorteilhafterweise kann die Hauptmagnetfeldeinheit mindestens einen Permanentmagnet oder mindestens eine Magnetspule ohne Kühlung aufweisen.

Dadurch wird kein Kühlsystem benötigt, so dass die MRT-Vorrichtung weniger aufwendig ist und dadurch kostengünstiger hergestellt werden kann.

Vorteilhafterweise kann die Patientenöffnung zusätzlich einen dritten Bereich mit einem dritten Querschnitt und einem dritten Innendurchmesser senkrecht zu einer Mittelachse der MRT-Vorrichtung aufweisen, der parallel zum ersten Innendurchmesser angeordnet ist, wobei der dritte Innendurchmesser größer als der zweite Innendurchmesser des zweiten Bereichs ist.

Der dritte Bereich kann unterhalb des zweiten Bereichs und unterhalb des ersten Bereichs angeordnet sein, so dass die Patientenöffnung wie eine Stufenpyramide geformt ist. Auf diese Weise kann der erste Bereich den Kopf des Patienten, der zweite Bereich den Schulterbereich des Patienten und der dritte Bereich den Bauchbereich und den Hüftbereich des Patienten umfassen.

Die Erfindung betrifft weiterhin ein Verfahren zur Positionierung eines Patienten relativ zu der oben genannten MRT-Vorrichtung, wobei an der MRT-Vorrichtung ein Patientensitz angebracht ist, wobei der Patientensitz Verstellmittel aufweist. Der Patient wird auf dem Patientensitz positioniert und wird in eine Aufnahmeposition gebracht, indem der Patient mittels der Verstellmittel entlang einer Fahrachse in die MRT-Vorrichtung zumindest teilweise hineingefahren wird, bis ein Kopf des Patienten zumindest teilweise im ersten Bereich der Patientenöffnung der MRT-Vorrichtung angeordnet ist.

Der Patientensitz ist an der MRT-Vorrichtung fest angebracht und ermöglicht mittels der Verstellmittel eine genaue Positionierung des Patienten, insbesondere des Kopfes des Patienten im ersten Bereich der Patientenöffnung der MRT-Vorrichtung.

Ein Vorteil dieses Verfahrens besteht darin, dass der Patient mittels der Verstellmittel in die MRT-Vorrichtung hineingefahren wird und insbesondere der Kopf des Patienten kontrolliert im ersten Bereich der MRT-Vorrichtung positioniert wird. Dadurch werden Positionierungsfehler des Patienten innerhalb der MRT-Vorrichtung verhindert, die beispielsweise durch eine fehlerhafte manuelle Bedienung verursacht werden können.

Die MRT-Vorrichtung ist schräg angeordnet, wobei die Fahrachse einen Winkel relativ zu der Mittelachse der MRT-Vorrichtung aufweisen kann, der innerhalb eines Toleranzwinkelbereichs zwischen +10° und -10° relativ zu der Mittelachse der MRT-Vorrichtung angeordnet ist.

Dadurch wird gewährleistet, dass der Patient mit der Innenwand der Patientenöffnung beim Hineinfahren des Patienten in die MRT-Vorrichtung nicht kollidiert.

Vorteilhafterweise können die Verstellmittel des Patientensitzes mechanisch durch einen Benutzer verstellt werden, um den Patientensitz in die Aufnahmeposition zu bringen.

Dadurch ist also kein Elektromotor oder eine elektronische Steuerung erforderlich, um den Patientensitz zu verstellen.

Vorteilhafterweise können die Verstellmittel des Patientensitzes durch mindestens einen Elektromotor angetrieben werden und mittels einer Steuerungseinheit angesteuert werden, um den Patientensitz in die Aufnahmeposition zu bringen.

Dadurch kann der Patientensitz automatisch verstellt werden, bis der Kopf des Patienten den ersten Bereich der Patientenöffnung erreicht.

### Kurzbeschreibung der Zeichnungen

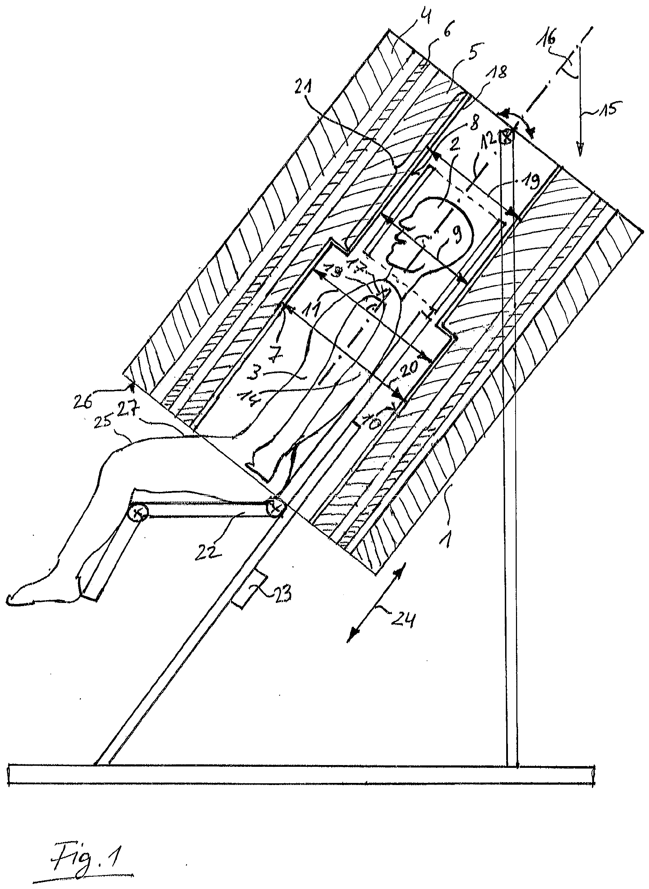

Die Erfindung wird anhand der Zeichnungen erläutert. Es zeigt, die
- Fig. 1: eine MRT-Vorrichtung zur Vermessung eines Kopfbereichs.

### Ausführungsbeispiele

Die Fig. 1 zeigt eine MRT-Vorrichtung 1 zur Vermessung eines Kopfbereichs 2 eines Patienten 3, umfassend mindestens eine Hauptmagnetfeldeinheit 4, mindestens eine Gradientenspulenanordnung 5, eine Hochfrequenzspule 6, die als eine Anregungsspule und als eine Empfangsspule dient und eine geschlossene Patientenöffnung 7. Die geschlossene Patientenöffnung 7 weist dabei einen ersten Bereich 8 mit einem ersten Innendurchmesser 9 und einen zweiten Bereich 10 mit einem zweiten Innendurchmesser 11 auf. Der erste Innendurchmesser 9 und der zweite Innendurchmesser 11 sind parallel zueinander und senkrecht zu einer Mittelachse 12 angeordnet, die durch eine Strichpunktlinie dargestellt ist. Die Mittelachse 12 ist zugleich eine Symmetrieachse der beiden zylinderförmigen Bereiche 8 und 10 der Patientenöffnung 7. Im dargestellten Fall ist der Patient 3 bereits innerhalb der MRT-Vorrichtung so positioniert, dass der Kopf 2 des Patienten 3 im ersten Bereich 8 und ein Schulterbereich 13 sowie teilweise ein Torsobereich 14 im zweiten Bereich 10 der Patientenöffnung 7 angeordnet sind. Die Mittelachse 12 der MRT-Vorrichtung 1, die auch einer Z-Richtung einer nicht dargestellten Z-Gradientenspule entspricht, weist relativ zu einer Richtung 15 der Erdgravitation einen Winkel 16 zwischen 20° und 75° auf. Im vorliegenden Fall sind der erste Bereich 8 und der zweite Bereich 10 der Patientenöffnung 7 zylinderförmig ausgeführt. Falls der zweite Bereich einen ellipsenförmigen Querschnitt aufweisen würde, würde der zweite Innendurchmesser des zweiten Bereichs entlang einer coronal angeordneten Schulterachse 17 des Schulterbereichs 13, die als ein Kreuz dargestellt ist, gemessen werden. Der erste Innendurchmesser 9 kann beispielsweise zwischen 250 mm und 650 mm und der zweite Innendurchmesser 11 zwischen 500 mm und 800 mm aufweisen. Die Gradientenspulenanordnung 5 ist im vorliegenden Fall unmittelbar hinter einer Verkleidung 18 der Patientenöffnung 7 angeordnet, so dass die Gradientenspulenanordnung 5 im ersten Bereich 8 einen kleineren Gradientenspulen-Innendurchmesser 19 als einen zweiten Gradientenspulen-Innendurchmesser 20 im zweiten Bereich 10 aufweist.

Eine Länge 21 des ersten Bereichs 8 der Patientenöffnung 7 weist mindestens 150 mm auf. An der MRT-Vorrichtung 1 ist ein Patientensitz 22 fest angebracht, wobei der Patientensitz 22 Verstellmittel 23 aufweist, die dem Patientensitz 22 entlang einer Fahrachse 24 verstellen können. Der Patient 3 wird also auf dem Patientensitz 22 positioniert und in die dargestellte Aufnahmeposition gebracht, in dem der Patient 3 mittels der Verstellmittel 23 entlang der Fahrachse 24 in die Patientenöffnung 7 der MRT-Vorrichtung 1 zumindest teilweise hineingefahren wird, bis der Kopf 2 des Patienten 3 zumindest teilweise im ersten Bereich 8 angeordnet ist. Die Verstellmittel 23 umfassen im vorliegenden Fall einen Elektromotor der den Patientensitz antreibt, wobei der Elektromotor mittels einer Steuerungseinheit entsprechend angesteuert wird, um den Patientensitz 22 in die Aufnahmeposition zu bringen. Die Ausrichtung der Fahrachse 24 ist im vorliegenden Fall parallel zur Mittelachse 12 der MRT-Vorrichtung 1 ausgestaltet. Ein Vorteil der schrägen Anordnung der MRT-Vorrichtung 1 besteht darin, dass die Kollisionswahrscheinlichkeit reduziert wird. Denn der Patient kann in einer nach hinten gelehnten, sitzenden Position auf einem Patientensitz positioniert werden und in die MRT-Vorrichtung hineingefahren werden. Durch die schräge Anordnung der MRT-Anordnung 1 werden also die Knie 25 nicht so stark angewinkelt, wie in einer aufrecht sitzenden Patientenposition, so dass die Kollision einer unteren Fläche 26 der MRT-Vorrichtung 1 mit Oberschenkeln 27 des Patienten 3 oder den Knien 25 des Patienten 3 beim Hineinfahren verhindert wird.

### Bezugszeichen

- 1: MRT-Vorrichtung
- 2: Kopfbereich
- 3: Patient
- 4: Hauptmagnetfeldeinheit
- 5: Gradientenspulenanordnung
- 6: Hochfrequenzspule
- 7: Patientenöffnung
- 8: erster Bereich
- 9: erster Innendurchmesser
- 10: zweiter Bereich
- 11: zweiter Innendurchmesser
- 12: Mittelachse
- 13: Schulterbereich
- 14: Torsobereich
- 15: Richtung
- 16: Winkel
- 17: Schulterachse
- 18: Verkleidung
- 19: Gradientenspulen-Innendurchmesser
- 20: zweiter Gradientenspulen-Innendurchmesser
- 21: Länge
- 22: Patientensitz
- 23: Verstellmittel
- 24: Fahrachse
- 25: Knie
- 26: untere Fläche
- 27: Oberschenkel

## Patentansprüche

1. MRT-Vorrichtung (1) zur Vermessung eines Kopfbereichs (2) eines Patienten (3), umfassend mindestens eine Hauptmagnetfeldeinheit (4), mindestens eine Gradientenspulenanordnung (5), eine geschlossene Patientenöffnung (7), mindestens eine Anregungsspule (6) und mindestens eine Empfangsspule, **dadurch gekennzeichnet, dass** eine geschlossene Patientenöffnung (7) der MRT-Vorrichtung (1) in einem ersten Bereich (8) einen ersten Innendurchmesser (9) senkrecht zu einer Mittelachse (12) der MRT-Vorrichtung (1) und im zweiten Bereich (10) einen zweiten Innendurchmesser (11) aufweist, der parallel zum ersten Innendurchmesser (9) angeordnet ist, wobei der erste Innendurchmesser (9) kleiner als der zweite Innendurchmesser (11) ist, wobei der erste Bereich (8) zumindest teilweise den Kopfbereich (2) des Patienten umfasst, wobei der zweite Bereich (10) zumindest teilweise einen Torsobereich (14) des Patienten umfasst, wobei die MRT-Vorrichtung (1) schräg angeordnet ist, wobei die Mittelachse (12) der MRT-Vorrichtung (1) relativ zu einer Richtung (15) der Erdgravitation einen Winkel (16) zwischen 20° und 75° aufweist.

2. MRT-Vorrichtung (1) nach Anspruch 1, **dadurch gekennzeichnet, dass** der zweite Innendurchmesser (11) des zweiten Bereichs (10) entlang einer coronal angeordneten Schulterachse (17) des Torsobereichs (14) des Patienten angeordnet ist.

3. MRT-Vorrichtung (1) nach Anspruch 1 oder 2, **dadurch gekennzeichnet, dass** ein erster Querschnitt der Patientenöffnung (7) im ersten Bereich (8) und/oder ein zweiter Querschnitt im zweiten Bereich (10) eine kreisförmige Form, eine elliptische Form oder eine rechteckige Form aufweist.

4. MRT-Vorrichtung (1) nach einem der Ansprüche 1 bis 3, **dadurch gekennzeichnet, dass** die Patientenöffnung (7) im ersten Bereich (8) den ersten Innendurchmesser (9) zwischen 250 mm und 650 mm und im zweiten Bereich (10) den zweiten Innendurchmesser (11) zwischen 500 mm und 800 mm aufweist.

5. MRT-Vorrichtung (1) nach einem der Ansprüche 1 bis 4, **dadurch gekennzeichnet, dass** die Gradientenspulenanordnung (5) innerhalb der MRT-Vorrichtung (1) um die Patientenöffnung (7) angeordnet ist, wobei die Gradientenspulenanordnung (5) im ersten Bereich (8) der Patientenöffnung (7) einen ersten Gradientenspulen-Innendurchmesser (19) und im zweiten Bereich (10) der Patientenöffnung (7) einen zweiten Gradientenspulen-Innendurchmesser (20) aufweist, der parallel zum ersten Gradientenspulen-Innendurchmesser (19) angeordnet ist, wobei der erste Gradientenspulen-Innendurchmesser (19) kleiner als der zweite Gradientenspulen-Innendurchmesser (20) ist.

6. MRT-Vorrichtung (1) nach einem der Ansprüche 1 bis 5, **dadurch gekennzeichnet, dass** die Gradientenspulenanordnung (5) nur im ersten Bereich (8), also umfassend den Kopfbereich (2) des Patienten, angeordnet ist und dass der zweite Bereich (10) umfassend den Torsobereich (14), frei von der Gradientenspulenanordnung (5) ist.

7. MRT-Vorrichtung (1) nach einem der Ansprüche 1 bis 6, **dadurch gekennzeichnet, dass** der erste Bereich (8) der Patientenöffnung (7) eine Länge von mindestens 150 mm aufweist.

8. MRT-Vorrichtung (1) nach einem der Ansprüche 1 bis 8, **dadurch gekennzeichnet, dass** die Gradientenspulenanordnung (5) eine Z-Gradientenspule für eine Z-Richtung aufweist, wobei die Z-Gradientenspule Spulenwindungen aufweist, die zylinderförmig aufgewickelt sind, wobei der Abstand der Spulenwindungen zueinander über die Länge der Z-Gradientenspule in Z-Richtung variiert.

9. MRT-Vorrichtung (1) nach Anspruch 8, **dadurch gekennzeichnet, dass** die Windungsdichte der Spulenwindungen der Z-Gradientenspule symmetrisch zu einer Ebene der Z-Gradientenspule ausgestaltet ist, die senkrecht zu der Mittelachse der MRT-Vorrichtung angeordnet ist.

10. MRT-Vorrichtung (1) nach Anspruch 8, **dadurch gekennzeichnet, dass** die Windungsdichte der Spulenwindungen der Z-Gradientenspule asymmetrisch ist, wobei die Spulenwindungen der Z-Gradientenspule auf einer zu einem Torso des Patienten gerichteten Seite im Vergleich zu der gegenüberliegenden zum Kopf des Patienten gerichteten Seite eine höhere Windungsdichte aufweisen.

11. Verfahren zur Positionierung eines Patienten relativ zu einer MRT-Vorrichtung (1) nach einem der Ansprüche 1 bis 10, **dadurch gekennzeichnet, dass** an der MRT-Vorrichtung (1) ein Patientensitz (22) angebracht ist, wobei der Patientensitz (22) Verstellmittel (23) aufweist, wobei der Patient (3) auf dem Patientensitz (22) positioniert wird und in eine Aufnahmeposition gebracht wird, indem der Patient (3) mittels der Verstellmittel (23) entlang einer Fahrachse (24) in die MRT-Vorrichtung (1) zumindest teilweise hineingefahren wird, bis ein Kopf (2) des Patienten (3) zumindest teilweise im ersten Bereich (8) der Patientenöffnung (7) der MRT-Vorrichtung (1) angeordnet ist.

12. Verfahren nach Anspruch 11, **dadurch gekennzeichnet, dass** die Verstellmittel (23) des Patientensitzes (22) mechanisch durch einen Benutzer verstellt werden, um den Patientensitz (22) in die Aufnahmeposition zu bringen.

13. Verfahren nach Anspruch 11, **dadurch gekennzeichnet, dass** die Verstellmittel (23) des Patientensitzes (22) durch mindestens einen Elektromotor angetrieben werden und mittels einer Steuerungseinheit angesteuert werden, um den Patientensitz (22) in die Aufnahmeposition zu bringen.
